# EUROPÄISCHE PATENTSCHRIFT

(11) **EP 0 529 500 B1**
(45) Veröffentlichungstag und Bekanntmachung des Hinweises auf die Patenterteilung: **09.11.1994**
(21) Anmeldenummer: 92114146.1
(22) Anmeldetag: 19.08.1992
(51) Int. Cl.: A61K 31/165, A61K 31/42

(54) **Arzneimittel zur Behandlung von Abstossungsreaktionen bei Organverpflanzungen**
Medicament for the treatment of rejection reactions during organ transplantations
Médicament pour le traitement de réactions de rejet lors de transplantations d'organes

(30) Priorität: 22.08.1991 DE 4127737
(43) Veröffentlichungstag der Anmeldung: 03.03.1993
(73) Patentinhaber: HOECHST AKTIENGESELLSCHAFT, 65926 Frankfurt am Main (DE)
(72) Erfinder: Bartlett, Robert Ryder, Dr., W-6100 Darmstadt (DE)

(56) Entgegenhaltungen:
- DE-A- 3 534 440
- TRANSPLANTATION PROCEEDINGS Bd. 24, Nr. 2, 1992, Seiten 718 - 719; K. ULRICHSET AL.: 'Suppression of Natural Xenophile Antibodies with the novelimmunomodulating drug Leflunomide'
- AGENTS AND ACTIONS Bd. 32, Nr. 1/2, 1991, Seiten 10 - 21; R.R.BARTLETT ET AL.:'Leflunomide (HWA 486), a novel immunomodulating compound for the treatment ofautoimmune disorders and reactions leading to transplantation rejection'
- TRANSPLANTATION PROCEEDINGS Bd. 23, Nr. 1, 1991, Seiten 1083 - 1086; C.C.A.KÜCHLE ET AL.: 'Prevention of kidney and skin graft rejection in rats by leflunomide, a new immunomodulating agent'
- KLIN. WOCHENSCHR. Bd. 68, Nr. S21, 1990, Seiten 15 - 25; D.B.J.HERRMANN ET AL.:'Drugs in autoimmune diseases'
- Transplantation, vol. 46, no. 1, 1988, pages 1-20

## Beschreibung

Aus der Europäischen Patentschrift 13 376 ist 5-Methylisoxazol-4-carbonsäure-(4-trifluormethyl)-anilid (Verbindung 1) als antiphlogistisch bekannt. Dort sind ebenfalls Verfahren zur Herstellung dieser Verbindung beschrieben.

Ferner ist bekannt, daß die Verbindung 1 und ihr Metabolit N-(4-Trifluormethylphenyl)-2-cyano-3-hydroxycrotonsäureamid (Verbindung 2) immunmodulierende Eigenschaften haben, so daß sie sich als Arzneimittel gegen chronische Graft-versus-Host-Krankheiten sowie gegen Autoimmunerkrankungen, insbesondere systemischen Lupus erythematodes eignen (EP-A-217 206).

Die US-Patentschrift 4 061 767 beschreibt die Verwendung von 2-Hydroxyethylidencyanessigsäureanilidderivaten zur Herstellung von Arzneimitteln mit antiphlogistischer und analgetischer Wirkung.

In der Publikation Agents and Actions (32, 1991, Seiten 10 bis 21) wird beschrieben, daß das Auftreten der akuten Abstoßungsreaktion durch Verbindung 1 und 2 verlangsamt wird. In der Publikation von Küchle et al. (Transplantation Proceedings, 23, 1991, Seiten 1083 bis 1086) wird die Wirkung der Verbindung 1 auf Abstoßungsreaktionen beschrieben, die später als 8 Tage nach Transplantation auftreten.

In den USA wurden 1990 15 000 Organverpflanzungen vorgenommen. Die Mehrzahl der Verpflanzungen betrifft die Niere, aber es werden auch zunehmend Herz, Haut, Lunge, Leber und Bauchspeicheldrüse verpflanzt. Bei einer Vielzahl der Patienten kommt es dabei zu einer Abstoßungsreaktion des Körpers gegen das übertragene Organ, das von einem anderen Menschen stammt. Man unterscheidet dabei unter drei Formen der Abstoßungsreaktion: hyperakute, akute und chronische Abstoßung.

Die hyperakute Abstoßung wird im wesentlichen durch im Blut zirkulierende Antikörper verursacht, die gegen das Gewebe des übertragenen Organs (Transplantat) gerichtet sind, und in sehr kurzer Zeit - häufig in Minuten - zu Nekrosen am Transplantat führen.

Bei der akuten Abstoßung des Transplantats ist die Abstoßungsreaktion zeitlich verzögert. Daneben gibt es die chronische Form des Krankheitsverlaufes. Die Transplantate überleben das erste Jahr der Verpflanzung, können jedoch im Laufe der nächsten Jahre abgestoßen werden. Ferner ist bekannt, daß sich die Transplantat-Wirt-Beziehung nicht allein auf die Abstoßung durch den Wirtsorganismus beschränkt; in bestimmten Fällen kann eine vom Transplantat ausgehende, gegen das Wirtsgewebe gerichtete Immunreaktion eintreten (EP-A-217 206). Man unterscheidet daher zwischen einer Abstoßungsreaktion zwischen Transplantat und Wirt und zwischen Wirt und Transplantat.

Ferner ist bekannt, daß es auch zu Abstoßungsreaktionen kommt, wenn Organe von verschiedenen Organismenarten, beispielsweise von Maus auf die Ratte, übertragen werden (Roitt et al., Immunology, Gower Medical Publishing Ltd., 1985).

Bisher sind keine Arzneimittel bekannt, die einen wirksamen Schutz gegen die hyperakute Abstoßungsreaktion bieten. In der Klinik werden bisher Spender und Empfänger des Organs auf Unverträglichkeit getestet. Bei 20 bis 40 % der Patienten ergibt sich dabei der Fall, daß sie kein Spenderorgan erhalten können. Die akute Abstoßungsreaktion kann behandelt werden, jedoch zeigen die Medikamente bei der Therapie nephrotoxische Nebenwirkungen. Bisher sind auch keine Medikamente bekannt, die die Ursache der chronischen Abstoßungsreaktion behandeln können.

Als wesentlicher pathogener Faktor für den Gewebsuntergang bei dem Transplantat werden allophile und xenophile Antikörper angesehen (Auchincloss H., Transplantation 46, 1, 1988). Diese Antikörper sind im wesentlichen für die Abstoßung bei Organübertragungen innerhalb einer Organismusart (allo) oder zwischen zwei verschiedenen Arten (xeno) verantwortlich.

Überraschenderweise zeigen Verbindung 1 und ihr Metabolit, die obengenannte Verbindung 2, eine starke Inhibierung der Bildung allophiler oder xenophiler Antikörper. Damit ergibt sich die Möglichkeit, die hyperakute, akute und chronische Abstoßungsreaktion des Empfängers gegen das verpflanzte Organ wirksam zu behandeln.

Die Erfindung betrifft daher die Verwendung von 5-Methyl-isoxazol-4-carbonsäure-(4-trifluormethyl-phenyl)-anilid und N-(4-Trifluormethylphenyl)-2-cyano-3-hydroxycrotonsäureamid und/oder ihre physiologisch verträglichen Salze zur Herstellung von Arzneimitteln zur Behandlung von hyperakuten Abstoßungsreaktionen des Organempfängers gegen das verpflanzte Organ.

Ferner betrifft die Erfindung die Verwendung der Verbindung der Formel 1 und/oder 2, wobei die Verbindung 2 als solche oder in Form eines physiologisch verträglichen Salzes vorliegt, zur Herstellung von Arzneimitteln zur Behandlung von Abstoßungsreaktionen des Organempfängers gegen das verpflanzte Organ, wobei das Organ von einer Organismusart auf eine andere Art übertragen wird.

Geeignete physiologisch verträgliche Salze der Verbindung 2 sind beispielsweise Alkali, Erdalkali- oder Ammoniumsalze, einschließlich solcher von physiologisch verträglichen organischen Ammoniumbasen.

Unter dem Begriff Organ, werden alle Organe bei Säugetieren, insbesondere des Menschen verstanden, beispielsweise Niere, Herz, Haut, Leber, Bauchspeicheldrüse, Muskel, Knochen, Darm oder Magen, aber auch Blut oder Haare. Unter Abstoßungsreaktion sind alle Abwehrmaßnahmen des Empfängerorganismusses gemeint, die letztlich zum Zell- oder Gewebsuntergang des übertragenen Organs führen bzw. die Lebenfähigkeit des übertragenen Organs beeinträchtigen.

Die Verbindungen 1 und 2 können nach folgendem Verfahren hergestellt werden:
Eine Verbindung der Formel I 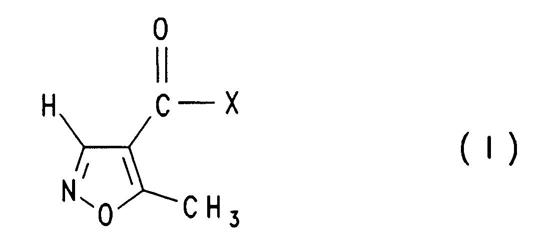
in der X für ein Halogenatom, vorzugsweise Chlor oder Brom steht, wird mit dem Amin der Formel II 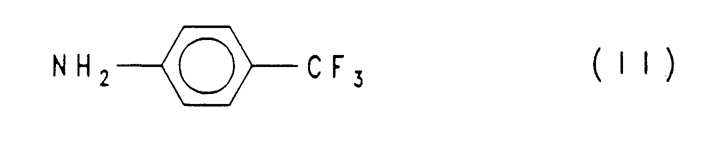
zur Verbindung 1, umgesetzt und kann anschließend in Gegenwart eines basischen Mittels zur Verbindung 2 umgesetzt werden.

Die obengenannten Umsetzungen erfolgen unter Standardbedingungen auf bekannt Weise (EP-B-13 376, US 4 061 767).

Die Ausgangsstoffe der Umsetzungen sind bekannt oder lassen sich nach literaturbekannten Methoden leicht herstellen.

Die Erfindung betrifft auch Arzneimittel, die eine wirksame Menge der Verbindung 1 oder Verbindung 2 und/oder physiologisch verträgliche Salze der Verbindung 2 enthalten, neben pharmazeutisch geeigneten und physiologisch verträglichen Trägerstoffen, Verdünnungsmitteln und/oder anderen Wirk- und Hilfsstoffen.

Die Erfindung betrifft auch ein Verfahren zur Herstellung eines Arzneimittels zur Behandlung von Abstoßungsreaktionen des Organempfängers gegen das verpflanzte Organ, das dadurch gekennzeichnet ist, daß man die Verbindung 1 oder 2 und/oder ein physiologisch verträgliches Salz der Verbindung 2 mit einem pharmazeutisch geeigneten und physiologisch annehmbaren Träger und gegebenenfalls weiteren geeigneten Wirk-, Zusatz- oder Hilfsstoffen in eine geeignete Darreichungsform bringt.

Die erfindungsgemäßen Arzneimittel können oral, topisch, rektal, intravenös oder auch parenteral appliziert werden. Die Applikation erfolgt vor, während und nach einer Organverpflanzung beim Empfänger und/oder Spender.

Geeignete feste oder flüssige galenische Darreichungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwas steriles Waser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis von der Verbindung 1 oder 2 und/oder physiologisch verträgliche Salze der Verbindung 2 enthält. Bei festen Dosierungseinheiten, wie Tabletten, Kapseln oder Suppositorien, kann diese Dosis bis zu etwa 300 mg, bevorzugt jedoch 10 bis 200 mg betragen.

Für die Behandlung eines Patienten (70 kg), dem ein Organ übertragen wurde sind in frühen Phasen nach der Übertragung eine intravenöse Infusionsbehandlung von maximal 1200 mg pro Tag und in der späteren Rehabilitationsphase eine orale Verabreichung von 3 mal 300 mg pro Tag der Verbindung 1 oder 2 und/oder der entsprechenden Salze der Verbindung 2 indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehreren kleineren Dosierungseinheiten als auch durch Mehrfachgabe unterteilter Dosen in bestimmten Intervallen erfolgen.

Schließlich können die Verbindung 1 oder 2 und/oder deren entsprechende Salze bei der Herstellung der vorgenannten galenischen Darreichungsformen auch zusammen mit anderen geeigneten Wirkstoffen, beispielsweise Antiuricopathika, Thrombocytenaggregationshemmern, Analgetika und steroidalen oder nichtsteroidalen Antiphlogistika, kombiniert werden.

### Beispiel 1

### Pharmakologische Prüfungen und Ergebnisse

2 bis 3 Monate alte Ratten (LEW) werden mit 2·10⁷ menschlichen periferen Blutlymphocyten intraperitoneal (i.p.) sensibilisiert. Die i.p. Applikation der Verbindung 2 beginnt 4 Tage vor der Sensibilisierung und endet 10 Tage nach der Sensibilisierung der Ratten. Serumproben werden aus der Schwanzvene entnommen, bei -80°C gelagert; durch Hitzedesaktivierung wird die Komplementfunktion vermieden.

Die natürlich vorkommenden xenophilen Antikörper (NXA) und die durch Sensibilisierung induzierten xenophilen Antikörper (SXA) werden titriert und lebenden menschlichen periferen Blutlymphocyten zugesetzt (45 min. bei 20°C). Nach intensivem Waschen werden FITC-markierte Ziegen-Antiratten-IgG oder -IgM-Antikörper zugefügt und die Bindung an sXA oder nXAdurch Flow-Cytometrie (FACScan, Becton Dickinson) quantitativ bestimmt.
A) Nicht-sensibilisierte Ratten
   Männliche LEW-Ratten enthalten in ihrem Serum NXA die an vitale menschliche perifere Blutlymphocyten binden und zwar meistens mit einem durchschnittlichen IgM-Titer von 1:4 und einem durchschnittlichen IgG-Titer der kleiner als 1:1 ist.
   Die Ratten (n=8), die jeden Tag mit 10mg/kg der Verbindung 2 behandelt werden, zeigen am 11. Tag eine 30 % Reduktion beim IgG-Titer und eine 50% Reduktion beim IgM-Titer.
B) Sensibilisierte Ratten
   Bei sensibilisierten Ratten ergeben sich stark erhöhte Mengen an SXA. Der IgG-Titer beträgt 1:1024 bis 1:16384 und der IGM-Titer 1:4 bis 1:256. Der IgG-Titer bleibt über 50 Tage stabil, während der IgM-Titer nach dem 10. Tag langsam abnimmt.

Die sensibilisierten Ratten (n=5) werden mit 3 oder 10 mg/kg der Verbindung 2 behandelt. Am 11. Tag ergeben sich folgende SXA-Titer:

| Verbindung (mg/kg) | IgG | IgM |
|---|---|---|
| 0 | 1:2048 | 1:90 |
| 3 | 1:25 | 1:16 |
| 10 | 1:1 | 1:1,5 |

Die Wirkung von Verbindung 2 auf die Bildung von allophilen Antikörper.

Männliche Brown-Norway-Ratten mit einem Gewicht von 200 bis 250 g werden als Spender von Herzen verwendet. Die Herzen werden in männliche Lewis-Ratten mit dem gleichen Gewicht transplantiert. Alle Ratten werden mit einer Standarddiät und Wasser versorgt und in einer Umgebung mit 12 Stunden Licht und Dunkel gehalten.

Den Empfängerratten (Lewis-Ratten) wird am Tag der Herztransplantation und dann im Abstand von 4 Tagen jeweils 1 ml Blut aus der Schwanzvene entnommen. Der Titer an allophilen Antikörpern im Blut der Empfängerratten wird dadurch bestimmt, daß verschiedene Verdünnungen des Empfängerbluts mit Milzzellen der Spenderratten (Brown-Norway-Ratten) und Kanninchen Komplement inkubiert werden. Die Cytotoxizität wird durch die Trypan-Blau-Ausschuß-methode bestimmt. Der pt-Wert für den Antikörper wird mit dem Standard-t-Test bestimmt, verglichen mit den nicht behandelten Kontrolltieren.

Die Behandlung mit der Verbindung 2 beginnt am 4. Tag nach der Transplantation. Es werden jeweils 10 mg/kg der Verbindung 2 einmal pro Tag i.p. verabreicht. Die Anzahl der Tiere, die gleichzeitig behandelt werden, ist 5 (n = 5). Die Behandlung endet am 17. Tag. Die nachfolgende Tabelle zeigt den Durchschnitt der Cytotoxizitätswerte (%) während der Behandlung.

| Serum Verdünnungen | Tage nach der Transplantation | | | | | | p-Wert am 17. Tag |
|---|---|---|---|---|---|---|---|
| | 0 | 4 | 7 | 11 | 14 | 17 | |
| 1:5 | 3 | 63 | 36 | 19 | 22 | 11 | 0,001 |
| 1:25 | 3 | 58 | 31 | 16 | 11 | 6 | 0,001 |
| 1:125 | 4 | 34 | 20 | 9 | 6 | 5 | 0,050 |

### Beispiel 2

### Herstellung von 5-Methylisoxazol-4-carbonsäure-(4-trifluormethyl)-anilid (Verbindung 1)

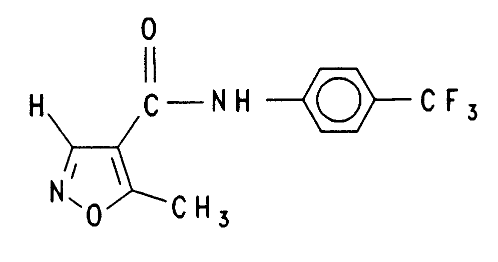

Eine Lösung von 0,05 Mol 5-Methylisoxazol-4-carbonsäurechlorid (7,3 g) in 20 ml Acetonitril werden tropfenweise bei Raumtemperatur in eine Lösung von 0,1 Mol 4-Trifluormethylanilin (16,1 g) in 150 ml Acetonitril gegeben. Nach 20 Minuten Rühren wird das ausgefallene 4-Trifluormethylanilin-hydrochlorid abgesaugt, zweimal mit je 20 ml Acetonitril gewaschen und die vereinigten Filtrate unter vermindertem Druck eingeengt. Man erhält so 12,8 g weißes, kristallines 5-Methylisoxazol-4-carbonsäure-(4-trifluormethyl)-anilid (Verbindung 1).

### Beispiel 3

### Herstellung von N-(4-Trifluormethylphenyl)-2-cyano-3-hydroxycrotonsäureamid (Verbindung 2)

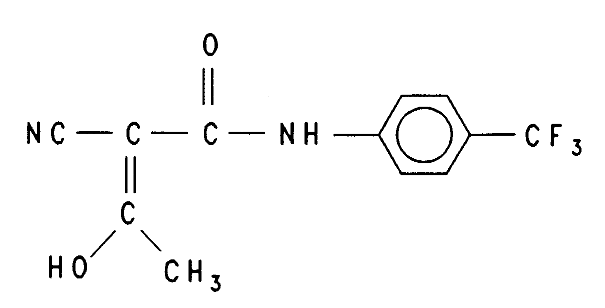

Es werden 0,1 Mol 5-Methylisoxazol-4-carbonsäure-(4-trifluormethyl)-anilid in 100 ml Methanol gelöst und bei + 10°C mit einer Lösung von 0,11 Mol (4,4 g) Natronlauge in 100 ml Wasser versetzt. Es wird 30 Minuten gerührt und nach Verdünnen mit Wasser mit konzentrierter Salzsäure angesäuert. Der ausgefallene Kristallbrei wird abgesaugt, mit Wasser gewaschen und an der Luft getrocknet.
Die Ausbeute beträgt 24,4 g N-(4-Trifluormethylphenyl)-2-cyano-3-hydroxycrotonsäureamid (Verbindung 2).
Schmelzpunkt aus Methanol 205 bis 206°C.

### Beispiel 4

### Akute Toxizität nach intraperitonealer Verabreichung

Die akute Toxizität nach intraperitonealer Verabreichung der Testsubstanzen wurde mit NMRI-Mäusen (20 bis 25 g) und SD-Ratten (120 bis 195 g) bestimmt. Die Testsubstanz wurde in einer 1 %igen Natrium-Carboxymethylcellulose-Lösung suspendiert. Die verschiedenen Dosierungen der Testsubstanz wurden den Mäusen in einem Volumen von 10 ml/kg Körpergewicht und den Ratten in einem Volumen von 5 ml/kg Körpergewicht verabreicht. Pro Dosierung wurden 10 Tiere verwendet. Nach 3 Wochen wurde die akute Toxizität nach der Methode von Litchfield und Wilcoxon bestimmt. Die Ergebnisse sind in der Tabelle zusammengefaßt.

**Tabelle**

| | Verbindung 1 akute Toxizität intraperitoneal LD₅₀ (mg/kg) | Verbindung 2 akute Toxizität intraperitoneal LD₅₀ (mg/kg) |
|---|---|---|
| NMRI-Maus | 185 (163 - 210) | 150 (100 - 200) |
| SD-Ratte | 170 (153 - 189) | |

## Patentansprüche

1. Verwendung der Verbindung der Formeln 1 und/oder 2 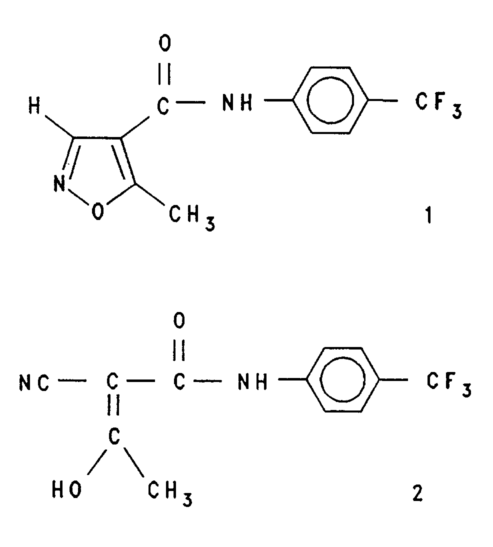 wobei die Verbindung 2 als solche oder in Form eines physiologisch verträglichen Salzes vorliegt, zur Herstellung von Arzneimitteln zur Behandlung von hyperakuten Abstoßungsreaktionen des Organempfängers gegen das verpflanzte Organ.

2. Verwendung nach Anspruch 1, dadurch gekennzeichnet, daß das Arzneimittel vor, während und/oder nach der Verpflanzung beim Empfänger oder Spender des Organs verabreicht wird.

3. Verwendung nach Anspruch 1 oder 2, dadurch gekennzeichnet, daß die Menge der allophilen oder xenophilen Antikörper vermindert wird.

4. Verwendung nach einem oder mehreren der Ansprüche 1 bis 3, dadurch gekennzeichnet, daß das Arzneimittel bei Säugetieren, insbesondere dem Menschen, eingesetzt wird.

5. Verwendung der Verbindung der Formeln 1 und/oder 2 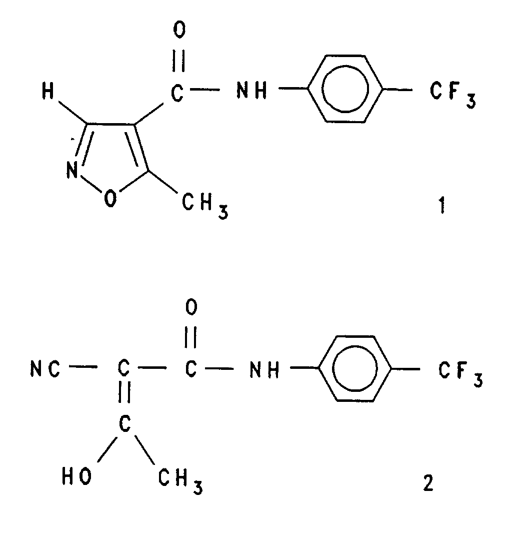 wobei die Verbindung 2 als solche oder in Form eines physiologisch verträglichen Salzes vorliegt, zur Herstellung von Arzneimitteln zur Behandlung von Abstoßungsreaktionen des Organempfängers gegen das verpflanzte Organ, wobei das Organ von einer Organismusart auf eine andere Art übertragen wird.

6. Verwendung nach Anspruch 5, dadurch gekennzeichnet, daß das Arzneimittel vor, während und/oder nach der Verpflanzung beim Empfänger oder Spender des Organs verabreicht wird.

7. Verwendung nach Anspruch 5 oder 6, dadurch gekennzeichnet, daß die Menge der allophilen oder xenophilen Antikörper vermindert wird.

8. Verwendung nach einem oder mehreren der Ansprüche 5 bis 7, dadurch gekennzeichnet, daß das Arzneimittel bei Säugetieren, insbesondere dem Menschen, eingesetzt wird.

## Claims

1. The use of the compound of the formulae I and/or II 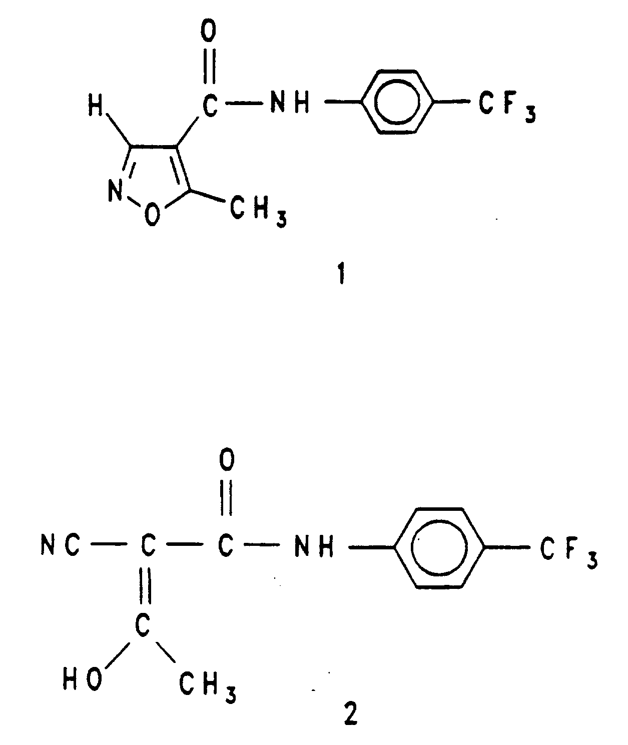 where compound 2 is present as such or in the form of a physiologically tolerable salt, for the preparation of pharmaceuticals for the treatment of hyperacute rejection reactions of the organ recipient to the transplanted organ.

2. The use as claimed in claim 1, wherein the pharmaceutical is administered to the recipient or donor of the organ before, during and/or after transplantation.

3. The use as claimed in claim 1 or 2, wherein the amount of allophilic or xenophilic antibodies is decreased.

4. The use as claimed in one or more of claims 1 to 3, wherein the pharmaceutical is employed in mammals, in particular the human.

5. The use of the compound of the formulae I and/or II 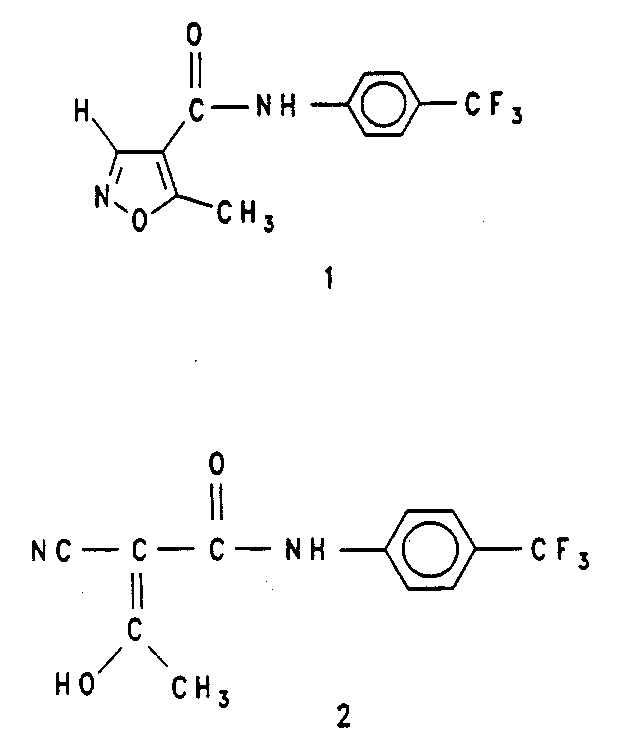 where compound 2 is present as such or in the form of a physiologically tolerable salt, for the preparation of pharmaceuticals for the treatment of rejection reactions of the organ recipient to the transplanted organ, the organ being transferred from one species of the organism to another species.

6. The use as claimed in claim 5, wherein the pharmaceutical is administered to the recipient or donor of the organ before, during and/or after transplantation.

7. The use as claimed in claims 5 or 6, wherein the amount of allophilic or xenophilic antibodies is decreased.

8. The use as claimed in one or more of claims 5 to 7, wherein the pharmaceutical is employed in mammals, in particular the human.

## Revendications

1. Utilisation du composé de formules 1 et/ou 2 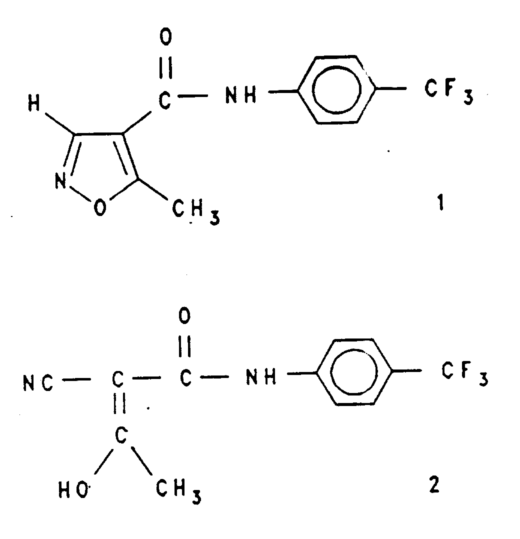 le composé 2 se trouvant tel quel ou sous forme d'un sel physiologiquement acceptable, pour la fabrication de médicaments pour le traitement de réactions de rejet hyperaiguës du récepteur d'organe contre l'organe transplanté.

2. Utilisation selon la revendication 1, caractérisée en ce que le médicament est administré au récepteur ou au donneur d'organe avant, pendant et/ou après la transplantation.

3. Utilisation selon la revendication 1 ou 2, caractérisée en ce que la quantité des anticorps allophiles ou xénophiles est diminuée.

4. Utilisation selon une ou plusieurs des revendications 1 à 3, caractérisée en ce que le médicament est utilisé chez des mammifères, l'homme en particulier.

5. Utilisation du composé de formules 1 et/ou 2 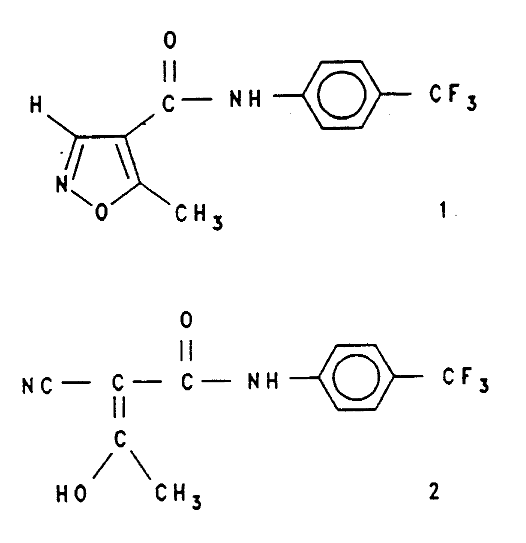 le composé 2 se trouvant tel quel ou sous forme d'un sel physiologiquement acceptable, pour la fabrication de médicaments pour le traitement de réactions de rejet du récepteur d'organe contre l'organe transplanté, l'organe étant transplanté d'une espèce d'organisme à une autre espèce.

6. Utilisation selon la revendication 5, caractérisée en ce que le médicament est administré au récepteur ou au donneur d'organe avant, pendant et/ou après la tranplantation.

7. Utilisation selon la revendication 5 ou 6, caractérisée en ce que la quantité des anticorps allophiles ou xénophiles est diminuée.

8. Utilisation selon une ou plusieurs des revendications 5 à 7, caractérisée en ce que le médicament est utilisé chez des mammifères, l'homme en particulier.
